(19) Europäisches Patentamt
European Patent Office
Office européen des brevets

(11) EP 2 447 244 B1

(12) **EUROPÄISCHE PATENTSCHRIFT**

(45) Veröffentlichungstag und Bekanntmachung des Hinweises auf die Patenterteilung:
**02.03.2016 Patentblatt 2016/09**

(51) Int Cl.:
***C07C 68/06*** *(2006.01)* ***C07C 68/08*** *(2006.01)*
***C07C 69/96*** *(2006.01)*

(21) Anmeldenummer: **11186426.0**

(22) Anmeldetag: **25.10.2011**

(54) **Verfahren zur kontinuierlichen Herstellung von Dialkylcarbonat**

Method for continuous production of dialkyl carbonate

Procédé destiné à la fabrication continue de carbonate de dialkyle

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.10.2010 DE 102010042936**

(43) Veröffentlichungstag der Anmeldung:
**02.05.2012 Patentblatt 2012/18**

(73) Patentinhaber: **Covestro Deutschland AG**
**51373 Leverkusen (DE)**

(72) Erfinder:
- **OOMS, Pieter**
  **47800 Krefeld (DE)**
- **RISSE, Friedhelm**
  **50739 Köln (DE)**
- **DÜX, Andre**
  **53332 Bornheim (DE)**
- **BUCHALY, Carsten**
  **40233 Düsseldorf (DE)**
- **PANCUR, Thomas**
  **24161 Altenholz (DE)**
- **SUSANTO, Arthur**
  **50670 Köln (DE)**
- **RONGE, Georg**
  **40549 Düsseldorf (DE)**
- **VANDEN EYNDE, Johan**
  **9052 Zwijnaarde (BE)**
- **WUYTACK, Wim**
  **9240 Zele (BE)**

(74) Vertreter: **Levpat**
**c/o Covestro AG**
**Alfred-Nobel-Straße 10**
**40789 Monheim am Rhein (DE)**

(56) Entgegenhaltungen:
**EP-A1- 1 961 731 EP-A1- 1 967 242**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 447 244 B1

## Beschreibung

Die vorliegende Erfindung betrifft ein kontinuierliches Verfahren zur Aufreinigung eines Dialkylcarbonat/Alkylalkohol-Gemisches bei der Herstellung von Dialkylcarbonat durch katalysierte Umesterung eines zyklischen Alkylencarbonates (z.B. Ethylen- oder Propylencarbonat) mit Alkylalkoholen. Für die Erhöhung der Produktqualität des Dialkylcarbonates ist die Wahl der Betriebsparameter der Dialkylcarbonat-Aufreinigungskolonne entscheidend, um die Bildung unerwünschter Nebenprodukte wie Alkoxyalkohole sowie aliphatische Carbonatether zu verringern.

Alkoxyalkohol entsteht aus der Reaktion von Alkylenoxid mit dem Alkylalkohol.

Der aliphatische Carbonatether entsteht aus der Reaktion des Alkoxyalkohol mit Dialhylcarbonat.

Dieser Carbonatether, der allgemein einen höheren Siedepunkt als der Alkylalkohol hat, verbleibt im Dialkylcarbonat. Wird das Dialkylcarbonat mit einer aromatischen Monohydroxyverbindung in einer weiteren Prozessstufe zu einem Diarylcarbonat umgesetzt, reagiert der aliphatische Carbonatether zu einem aromatischen Carbonatether weiter. In der anschließenden Umsetzung des Diarylcarbonates mit einer aromatischen Dihydroxyverbindung zu einem aromatischen Polycarbonat führt der aromatische Carbonatether zu einer Verschlechterung der Produkteigenschaften des Polycarbonates, wobei sowohl das Molekulargewicht, welches, gleiche Reaktionsbedingungen vorausgesetzt, in Gegenwart des aromatischen Carbonatethers geringer ausfällt als bei seiner Abwesenheit, als auch die Farbe des Polymers negativ beeinflusst werden.

Die Herstellung von Dialkylcarbonaten aus zyklischem Alkylencarbonat und Alkylalkohol, bei der gleichzeitig Alkylenglykol als Nebenprodukt entsteht, ist bekannt und vielfach beschrieben worden. In US 6 930 195 B2 wurde diese katalysierte Umesterungsreaktion als zweistufige Gleichgewichtsreaktion beschrieben. In der ersten Reaktionsstufe reagiert das zyklische Alkylencarbonat mit Alkohol zu Hydroxyalkylcarbonat als Zwischenprodukt. Das Zwischenprodukt wird dann in der zweiten Reaktionsstufe mit Hilfe von Alkohol umgesetzt zu den Produkten: Dialkylcarbonat und Alkylenglykol.

Für die technische Realisierung des Dialkylcarbonat-Herstellungsverfahrens hat sich die Verwendung einer reaktiven Destillationskolonne (im Folgenden auch Umesterungskolonne genannt), die unter anderem bereits in EP 530 615 A1, EP 569 812 A1 und EP 1 086 940 A1 beschrieben wurde, als besonders günstig erwiesen. In EP 569 812 A1 wird das zyklische Alkylencarbonat in den oberen Teil der Umesterungskolonne und der Dialkylcarbonat enthaltende Alkylalkohol in den mittleren oder unteren Teil der Umesterungskolonne kontinuierlich eingeführt. Zusätzlich wird unterhalb der Einführung des Dialkylcarbonat enthaltenden Alkylalkohols nahezu reiner Alkylalkohol eingeführt.

Eine Substanz wird als nahezu rein im Sinne dieser Erfindung bezeichnet, wenn sie weniger als 2 Gew.%, bevorzugt weniger als 1 Gew.% Verunreinigungen enthält. Das Schwersiedergemisch, welches das hergestellte Alkylenglykol als Nebenprodukt beinhaltet, wird am Sumpf der Umesterungskolonne kontinuierlich abgezogen. Das Leichtsiedergemisch, welches das hergestellte Dialkylcarbonat beinhaltet, wird am Kopf der Umesterungskolonne als Dialkylcarbonat-Alkylalkohol-Gemisch abgezogen und einem weiteren Aufreinigungsschritt unterworfen.

Die Destillationskolonne für die Aufreinigung des Dialkylcarbonat-Alkylalkohol-Gemisches wird bei einem höheren Druck betrieben, so dass ein weiteres Dialkylcarbonat-Alkylalkohol-Gemisch mit einem niedrigeren Dialkylcarbonat-Anteil am Kolonnenkopf abgezogen werden kann. Das Dialkylcarbonat als Hauptprodukt wird am Sumpf dieser Aufreinigungskolonne gewonnen.

Für die Entwicklung eines wirtschaftlich attraktiven Herstellungsverfahrens für Dialkylcarbonate spielen viele Faktoren eine wichtige Rolle. Die meisten Literaturquellen beschäftigen sich mit den Reaktionsparametern, wie z.B. Umsatz, Selektivität, Produktreinheit oder der Energieeffizienz des Verfahrens (z.B. EP 1 760 059 A1, EP 1 967 242 A1, EP 1 967 508 A1). Seltener werden die Einflussfaktoren zur Bildung von Nebenprodukten in der Dialkylcarbonat-Aufreinigungskolonne untersucht, obwohl diese Faktoren nicht unerheblich zu der wirtschaftlichen Attraktivität des Verfahrens beitragen. Daher werden in dieser Erfindung Maßnahmen eingeführt, um die Nebenproduktbildung in der Dialkylcarbonat-Aufreinigungskolonne zu erniedrigen.

In EP 1 760 059 A1 wird ein Verfahren zur Herstellung von Dialkylcarbonat und Alkylenglykol aus Alkylencarbonat und Alkylalkohol unter Verwendung eines homogenen Katalysators beschrieben. Die Reaktion findet in einer Destillationskolonne (Umesterungskolonne) statt. Am Kopf der Kolonne wird ein Gemisch bestehend aus Dialkylcarbonat und Alkylalkohol entnommen und einer Destillationskolonne zur Auftrennung dieses Gemisches (Dialkylcarbonat-Aufreinigungskolonne) zugeführt. Im Sumpf dieser Kolonne wird wiederum gereinigtes Dialkylcarbonat abgezogen. Dieses Dialkylcarbonat enthält einen aliphatischen Carbonatether, der von der Konzentration an Alkylenoxid im Alkylencarbonat, das der Umesterungskolonne zugeführt wird, abhängt. Das Alkylencarbonat wurde durch die Reaktion von Alkylenoxid mit Kohlenstoffdioxid hergestellt. Am Ende des Prozesses zur Herstellung von Alkylencarbonat ist festzustellen, dass das Alkylencarbonat noch geringe Mengen an Alkylenoxid enthält. In dem Verfahren zur Herstellung von Dialkylcarbonat und Alkylenglykol zeigt sich nun, dass, je mehr Alkylenoxid im Alkylencarbonat enthalten ist, desto größer ist die Konzentration des aliphatischen Carbonatethers im gereinigten Dialkylcarbonat.

Es stellt sich heraus, dass die Konzentration des aliphatischen Carbonatethers im gereinigten Dialkylcarbonat nur durch die Reduzierung des Gehaltes von Alkylenoxid im Alkylencarbonat verringert werden kann, die apparativ

aufwändig in der Herstellung des Alkylencarbonates gewährleistet werden muss, wie z. B. durch die Verwendung eines Nachreaktors oder einer zusätzlichen Destillation. Es bestand demnach Bedarf an einem Verfahren zur Reinigung von Dialkylcarbonat, welches geeignet ist, bei gleichbleibendem Reinheitsgrad des Dialhylcarbonates den Gehalt an aliphatischem Carbonatether ohne zusätzlichen apparativen Aufwand zu verringern.

EP 1 961 731 betrifft ein Verfahren zur Herstellung von Dialkylcarbonat durch Reaktion zwischen Diethylencarbonat und Methanol. Die Verweilzeit des Dialkylcarbonates in der Kolonne liegt im Bereich von 0,1 von 20 h.

Die Aufgabe, die der Erfindung zugrunde lag, bestand demnach darin, ein Verfahren zur Aufreinigung von Dialkylcarbonaten bereitzustellen, welches gegenüber bekannten Verfahren zu einem geringeren Gehalt an aliphatischem Carbonatether im gereinigten Dialkylcarbonat führt. Überraschend wurde nun gefunden, dass der Gehalt an Nebenprodukten im gereinigten Dialkylcarbonat, insbesondere Alkoxyalkohole sowie aliphatische Carbonatether, durch eine geeignete Wahl des Bereichs der mittleren Verweilzeit der Stoffe in der Dialkylcarbonat-Aufreinigungskolonne(n) reduziert werden kann.

Die mittlere Verweilzeit $t_{vwz}$ der flüssigen Phase in der Dialkylcarbonat-Aufreinigungskolonne(n) beträgt bevorzugt 0,3 bis 2 h und besonders bevorzugt 0,5 bis 2 h. Dabei ist die mittlere Verweilzeit $t_{vwz}$ definiert durch Formel (I):

$$t_{VWZ} := V \bullet \rho / (dm/dt_{DAC}) \qquad \text{(I)}$$

wobei:

| | |
|---|---|
| V:= | Volumen des Flüssigkeit-Holdups der Destillationskolonne unterhalb der Zuführstelle des Dialkylcarbonat-Alkohol-Gemisches |
| ρ:= | mittlere Dichte des Flüssigkeit-Holdups der Destillationskolonne unterhalb der Zuführstelle des Dialkylcarbonat-Alkohol-Gemisches |
| $dm/dt_{DAC}$:= | dem Sumpf der Destillationskolonne entnommener Massestrom, der das gereinigte Dialkylcarbonat enthält. |

Im Rahmen der Erfindung gereinigte Dialkylcarbonate sind bevorzugt solche der allgemeinen Formel (II)

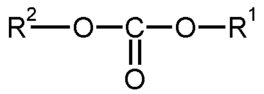
(II),

wobei $R^1$ und $R^2$ unabhängig voneinander für lineares oder verzweigtes, substituiertes oder unsubstituiertes $C_1$-$C_6$-Alkyl, bevorzugt für $C_1$-$C_4$-Alkyl stehen. Dabei können $R^1$ und $R^2$ gleich oder verschieden sein. Bevorzugt sind $R^1$ und $R^2$ gleich.

**$C_1$-$C_4$-Alkyl** steht im Rahmen der Erfindung beispielsweise für Methyl, Ethyl, n-Propyl, iso-Propyl, n-Butyl, sec.-Butyl, tert.-Butyl, **$C_1$-$C_6$-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Die Dialkylcarbonate werden bevorzugt aus zyklischen Alkylencarbonaten mit der Formel (III) hergestellt:

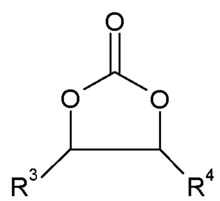
(III),

wobei in der Formel $R^3$ und $R^4$ unabhängig voneinander Wasserstoff, substituiertes oder nicht substituiertes **$C_1$-$C_4$-Alkyl**, substituiertes oder nicht substituiertes **$C_2$-$C_4$-Alkenyl** oder substituiertes oder nicht substituiertes **$C_6$-$C_{12}$-Aryl** und $R^3$ und $R^4$ gemeinsam mit den beiden Fünfring-C-Atomen einen gesättigten carbozyklischen Ring mit 5 - 8 Ringgliedern bedeuten können.

Bevorzugte Alkylencarbonate sind Ethylencarbonat und Propylencarbonat.

Die zyklischen Alkylencarbonate werden mit Alkoholen der Formel (IV)

$R^5$-OH (IV)

umgesetzt, wobei $R^5$ ein geradkettiges oder verzweigtes **$C_1$-$C_4$-Alkyl** bedeutet.

Bevorzugte Alkohole sind Methanol und Ethanol.

Alkylenoxide im Sinne des Verfahrens sind Verbindungen gemäß Formel (V)

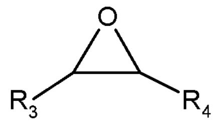

(V)

wobei $R^3$ und $R^4$ die gleiche Bedeutung besitzen wie oben.

Die Destillationskolonne zur Aufreinigung des Dialkylcarbonats verfügt bevorzugt über einen Verstärkungsteil mit bevorzugt 5 bis 40 theoretischen Stufen zur Aufkonzentrierung des Alkylalkohols und einen Abtriebsteil mit bevorzugt 5 bis 40 theoretischen Stufen zur Aufkonzentrierung des Dialkylcarbonates.

In den Kolonnensektionen können in allen Abschnitten der Dialkylcarbonat-Aufreinigungskolonne, d.h. sowohl in Verstärkungs- und gegebenenfalls Abtriebsteil Füllkörper oder strukturierte Packungen zum Einsatz kommen. Die zu verwendenden Füllkörper bzw. strukturierten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig-, Pall- und Navoloxringe, Interpackkörper, Berl-, Intalex- oder Torussättel genannt. Beispiele für strukturierte Packungen sind Blech- und Gewebepackungen (wie z.B. BX-Packungen, Montz Pak, Mellapak, Melladur, Kerapak und CY-Packung). Die verwendeten Füllkörper und/oder strukturierten Packungen können dabei aus verschiedenen Materialien, wie z.B. Glas, Steinzeug, Porzellan, Edelstahl, Kunststoff hergestellt sein.

Alternativ eignen sich auch dem Fachmann bekannte für Destillationen übliche Kolonnenböden, wie sie beispielsweise in Henry Z. Kister, "Distillation - Design", S. 259 off. beschrieben sind. Als Beispiele für Kolonnenböden seien Sieb-, Glocken-, Ventil- und Tunnelböden genannt.

Bevorzugt sind Füllkörper und strukturierte Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Rombopak, Kerapak und CY-Packungen. Die genauere Auslegung des zu verwendendes Abtriebsteils und des Verstärkerteils kann vom Fachmann vorgenommen werden.

Die Dimensionierung des Kolonnensumpfes erfolgt nach allgemeinen, dem Fachmann bekannten, Regeln. Alternativ zu einer standardmäßigen Ausführung des Kolonnensumpfes können Gestaltungsmaßnahmen zur Verringerung des Flüssigkeitsinhaltes erfolgen. Beispielsweise kann eine Einengung des Sumpfdurchmessers im Vergleich zum Kolonnenkörper realisiert oder eine geeignete Prallvorrichtung zur Verbesserung des Entgasungsvorganges der Flüssigkeit im Kolonnensumpf eingebaut und damit eine geringere Verweilzeit der Flüssigkeit eingestellt werden. Weiterhin kann durch die Realisierung eines geeigneten Zwangsumlaufverdampfersystems eine Einstellung der Verweilzeit der Flüssigkeit im Kolonnensumpf erfolgen. Darüber hinaus sind weiter Maßnahmen zur Verringerung des Flüssigkeitsinhaltes im Kolonnensumpf, wie z. B. die Einbringung geeigneter Verdrängungskörper, denkbar.

Die Trennung des Dialkylcarbonats und des Alkylalkohols erfolgt bevorzugt destillativ in einer oder mehreren Destillationskolonnen oder in einer Kombination aus Destillation und Membrantrennung - im Folgenden als Hybridprozess - bezeichnet (siehe z. B. US-4,162,200 A, EP 581 115 B1, EP 592 883 B1 und WO 2007/096343A1).

Bilden Alkylalkohol und Dialkylcarbonat ein Azeotrop (z.B. Methanol und Dimethylcarbonat) so kann auch ein zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet werden. Besonders bevorzugt wird das Zweidruckverfahren oder ein Hybridverfahren angewendet.

Ganz besonders bevorzugt wird die Trennung des Dialkylcarbonats und des Alkylalkohols - selbst in dem Falle, dass das Dialkylcarbonat und der Alkylalkohol ein Azeotrop bilden - in einer einzelnen Destillationskolonne durchgeführt. Diese Destillationskolonne wird bei einem Druck betrieben, der höher ist als der Druck der Umesterungskolonne(n). Der Betriebsdruck der Destillationskolonne liegt im Bereich von 1 bis 50 bar, vorzugsweise von 2 bis 20 bar. Am Sumpf der Destillationskolonne wird das nahezu reine Dialkylcarbonat entnommen und am Kopf ein Gemisch aus Dialkylcarbonat

und Alkylalkohol. Dieses Gemisch wird der oder den Umesterungskolonne(n) ganz oder teilweise zugeführt. Wird das Verfahren zur Herstellung von Dialkylcarbonat mit einem Verfahren zur Herstellung von Diarylcarbonat, welches durch Umesterung dieses Dialkylcarbonates mit einer aromatischen Hydroxyverbindung gebildet wird, gekoppelt, so kann ein Teil des Gemisches aus Dialkylcarbonat und Alkylalkohol, welches am Kopf der Destillationskolonne entnommen wird, einem entsprechenden Aufarbeitungsschritt für Alkylalkohol und Dialkylcarbonat in der Verfahrensstufe zur Herstellung von Diarylcarbonat zugeführt werden.

In einer besonders bevorzugten Ausführung, wenn das Dialkylcarbonat und der Alkylalkohol ein Azeotrop bilden, ist dieser Aufarbeitungsschritt ein Zweidruckverfahren. Solche Verfahren sind dem Fachmann grundsätzlich bekannt (vgl. z.B. Ullmann's Encyclopedia of Industrial Chemistry, Vol. 7, 2007, Kap. 6.4. und 6.5.; Chemie Ingenieur Technik (67) 11 / 95).

Bilden Alkylalkohol und Dialkylcarbonat ein Azeotrop, so weist das Destillat einer ersten Destillationskolonne des Verfahrensschritts zur Trennung von Dialkylcarbonat und Alkylalkohol vorzugsweise eine nahezu azeotrope Zusammensetzung auf. In diesem Fall wird dieses bevorzugt in einem Zweidruckverfahren wenigstens einer weiteren Destillationskolonne zugeführt, die bei einem Betriebsdruck arbeitet, der unter dem der ersten Destillationskolonne liegt. Durch den unterschiedlichen Betriebsdruck verschiebt sich die Lage des Azeotrops hin zu geringeren Anteilen an Alkylalkohol. Man erhält als Sumpfprodukt dieser zweiten oder weiteren Destillationskolonne(n) Alkylalkohol in einer Reinheit von 90 bis 100 Gew.-%, bezogen auf das Gesamtgewicht des isolierten Sumpfproduktes, und als Destillat ein nahezu azeotropes Gemisch. Die bei geringerem Betriebsdruck arbeitende zweite oder weitere(n) Destillationskolonne(n) wird in ganz besonders bevorzugten Ausführungsformen vorzugsweise mit der Kondensationswärme des oder der Kopfkondensator(en) der ersten Destillationskolonne betrieben.

Beim Zweidruckverfahren macht man sich die Druckabhängigkeit der azeotropen Zusammensetzung eines Zweistoffgemisches zunutze. Bei einem Gemisch aus Alkylalkohol und Dialkylcarbonat, wie beispielsweise Methanol und Dimethylcarbonat, verschiebt sich die azeotrope Zusammensetzung mit zunehmendem Druck zu höheren Alkylalkoholgehalten. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltene azeotrope Gemisch wird einer weiteren Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Alkylalkoholgehalten. Hierdurch ist es möglich, das in der Dialkylcarbonatkolonne erhaltene azeotrope Gemisch in ein Destillat mit nahezu azeotroper Zusammensetzung und nahezu reinen Alkylalkohol zu trennen. Das Destillat der Alkylalkoholkolonne wird wieder der Dialkylcarbonatkolonne an geeigneter Stelle zugeführt.

Der Betriebsdruck der Alkylalkoholkolonne wird vorzugsweise so gewählt, dass man diese mit der Abwärme der Dialkylcarbonatkolonne betreiben kann. Der Betriebsdruck liegt dabei von 0,1 bis 1 bar vorzugsweise von 0,3 bis 1 bar. Der Betriebsdruck der Dialkylcarbonatkolonne liegt im Bereich von 1 bis 50 bar, vorzugsweise von 2 bis 20 bar.

Eine beispielhafte Reaktionsführung bei der Trennung von Dialkylcarbonat und Alkylalkohol nach dem Zweidruckverfahren ist in Fig. 1 gezeigt.

Ein weiteres bevorzugtes Verfahren zur Trennung von Azeotropen aus Alkylalkohol und Dialkylcarbonat ist das Hybridverfahren. Beim Hybridverfahren erfolgt die Trennung eines Zweistoffgemisches mittels einer Kombination aus Destillation und Membranverfahren. Dabei macht man sich die Tatsache zunutze, dass man die Komponenten aufgrund ihrer polaren Eigenschaften und ihres unterschiedlichen Molekulargewichts mittels Membranen zumindest teilweise voneinander trennen kann. Im Falle eines Gemisches aus Alkylalkohol und Dialkylcarbonat, wie beispielsweise Methanol und Dimethylcarbonat, erhält man bei Verwendung geeigneter Membranen mittels Pervaporation oder Dampfpermeation ein alkylalkoholreiches Gemisch als Permeat und ein an Alkylalkohol verarmtes Gemisch als Retentat. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechenden azeotropen Zusammensetzung liegt, so erhält man als Destillat ein Gemisch mit im Vergleich zum Zulauf deutlich erhöhtem Alkylalkoholgehalt und als Sumpfprodukt nahezu reines Dialkylcarbonat.

Im Falle eines Hybridverfahrens aus Destillation und Dampfpermeation wird das Destillat der Kolonne dampfförmig entnommen. Das so erhaltene dampfförmige Gemisch wird gegebenenfalls nach Überhitzung einer Dampfpermation zugeführt. Diese wird so betrieben, dass man auf der Retentatseite nahezu den Betriebsdruck der Kolonne und auf der Permeatseite einen geringeren Druck einstellt. Der Betriebsdruck der Kolonne liegt dabei im Bereich von 1 bis 50 bar, bevorzugt von 1 bis 20 und besonders bevorzugt von 2 bis 10 bar. Der Druck auf der Permeatseite liegt von 0,05 bis 2 bar. Dabei erhält man auf der Permeatseite eine Alkylalkohol-reiche Fraktion mit einem Alkylalkoholgehalt von mindestens 70 Gew.-%, bevorzugt mindestens 90 Gew.%, bezogen auf das Gesamtgewicht der Fraktion. Das Retentat, welches einen im Vergleich zum Destillat der Kolonne reduzierten Alkylalkoholanteil enthält, wird gegebenenfalls kondensiert und wieder der Destillationskolonne zugeführt.

Im Falle eines Hybridverfahrens aus Destillation und Pervaporation wird das Destillat der Kolonne flüssig

entnommen. Das so erhaltene Gemisch wird gegebenenfalls nach Erhitzung einer Pervaporation zugeführt. Diese wird so betrieben, dass man auf der Retentatseite einen im Vergleich zur Kolonne identischen oder erhöhten Betriebsdruck und auf der Permeatseite einen geringeren Druck einstellt. Der Betriebsdruck der Kolonne liegt dabei im Bereich von 1 bis 50 bar, bevorzugt von 1 bis 20 und besonders bevorzugt von 2 bis 10 bar. Der Druck auf der Permeatseite liegt von 0,05 bis 2 bar. Dabei erhält man auf der Permeatseite eine Alkylalkohol-reiche dampfförmige Fraktion mit einem Alkylalkoholgehalt von mindestens 70 Gew.%, bevorzugt mindestens 90 Gew.%, bezogen auf das Gesamtgewicht der Fraktion. Das flüssige Retentat, welches einen im Vergleich zum Destillat der Kolonne reduzierten Alkylalkoholanteil enthält, wird wieder der Destillationskolonne zugeführt. Durch die Verdampfung des Permeats wird Wärme benötigt, die gegebenenfalls nicht in ausreichendem Maße im Zulaufstrom zur Pervaporation enthalten ist. Daher kann eine Membrantrennung mittels Pervaporation gegebenenfalls mit zusätzlichen Wärmetauschern beheizt werden, wobei diese integriert oder gegebenenfalls zwischen mehreren hintereinander geschalteten Pervaporationsschritten angebracht sind.

Die Trennung von Dialkylcarbonat und Alkylalkohol erfolgt im Falle eines Hybridverfahrens besonders bevorzugt mittels einer Kombination aus Destillation und Dampfpermeation.

Die zur Trennung von Alkylalkohol und Dialkylcarbonat erforderliche Wärme wird bei einer Temperatur von 100°C bis 300°C, vorzugsweise von 100°C bis 230°C, und besonders bevorzugt von 120°C bis 210°C, insbesondere besonders bevorzugt von 140°C bis 190°C zugeführt.

Das Herstellungsverfahren des Dialkylcarbonats kann kontinuierlich oder diskontinuierlich durchgeführt werden. Bevorzugt ist eine kontinuierliche Fahrweise.

Im Verfahren werden die zyklische(n) Alkylencarbonatverbindung(en) und der oder die Alkohol(e) bevorzugt im Molverhältnis von 1 : 0.1 bis 1 : 40, besonders bevorzugt von 1 : 1.0 bis 1 : 30, ganz besonders bevorzugt von 1 : 2.0 bis 1 : 20 eingesetzt. Dabei berücksichtigt das angegebene Molverhältnis nicht die Rückführung von zyklischer Alkylencarbonatverbindung oder Alkohol in die Umesterungskolonne über einen oder mehrere Kopfkondensator(en) oder einen oder mehrere etwaig vorhandene(n) Sumpfverdampfer.

Der Katalysator wird bevorzugt zusammen mit dem Strom enthaltend das zyklische Alkylencarbonat in gelöster oder suspendierter Form in die Umesterungskolonne über eine Einführungsstelle, die oberhalb der Einführungsstellen des Alkohols angeordnet ist, eingebracht. Alternativ kann der Katalysator auch separat beispielsweise im Alkohol, im Alkylenglykol oder in einem geeigneten inerten Lösungsmittel gelöst, zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Das Herstellungsverfahren von Dialkylcarbonat wird in einer Umesterungskolonne durchgeführt. In bevorzugten Ausführungsformen des Herstellungsverfahren kann der am Sumpf dieser Umesterungskolonne entnommene Flüssigkeitsstrom - gegebenenfalls nach Aufkonzentrierung - in einem oder mehreren weiteren Schritten einer weiteren Reaktion und/oder Reinigung unterzogen werden. Bevorzugt können einzelne oder alle solche weiteren Schritte in einer oder mehreren weiteren Kolonnen erfolgen.

Als Umesterungskolonne oder gegebenenfalls zweite bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die Umesterungskolonne enthält bevorzugt wenigstens einen Verstärkungsteil im oberen Teil der Kolonne und wenigstens eine Reaktionszone unterhalb des Verstärkungsteils. Der Verstärkungsteil weist bevorzugt 0 bis 30, bevorzugt 0,1 bis 30 theoretische Stufen auf.

In bevorzugten Ausführungsformen weist die Umesterungskolonne unterhalb einer Reaktionszone wenigstens einen Abtriebsteil auf.

Die Umesterungskolonne kann weiterhin bevorzugt mit einem oder mehreren Sumpfverdampfer(n) ausgestattet sein. Bei Ausführung der Umesterungskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die Umesterungskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die Umesterungskolonne zurückgeführt.

Der oder die Verstärkungsteil(e) können in bevorzugten Ausführungsformen zusammen mit dem oder den Reaktionsteil(en) und gegebenenfalls wenigstens einem Abtriebsteil in der Umesterungskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung des Alkylencarbonats bzw. Alkylenglykols stattfindet.

Unterhalb der Reaktionszone und eines gegebenenfalls vorhandenen Abtriebsteils wird ein Gemisch enthaltend Alkylenglykol, überschüssiges oder nicht umgesetztes Alkylencarbonat, Alkohol, Dialkylcarbonat, Umesterungskatalysatoren und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Alkohol reduziert, wobei in Anwesenheit des Umesterungskatalysators unter Umständen weiteres Dialkylcarbonat und Alkylenglykol gebildet werden. Die hierzu erforderliche Energie, wird bevorzugt durch einen oder mehrere Verdampfer zugeführt.

In allen Abschnitten der Umesterungskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl" Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novaloxringe, Berlsättel, BX-Packungen, Montz Pak, Mellapak, Melladur, Kerapak und CY-Packungen.

Alternativ eignen sich auch Kolonnenböden, wie beispielsweise Sieb-, Glocken-, Ventil-, Tunnelböden. In der oder den Reaktionszone(n) der Umesterungskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendenden Abtriebsteils und des oder der Verstärkerteil(e) kann vom Fachmann vorgenommen werden.

Die Temperatur der Reaktionszone(n) liegt bevorzugt im Bereich von 20 bis 200°C, besonders bevorzugt von 40 bis 180°C, ganz besonders bevorzugt von 40 bis 160°C. Es ist von Vorteil, die Umesterung nicht nur bei Normaldruck, sondern auch bei erhöhtem oder erniedrigtem Druck durchzuführen. Der Druck der Reaktionszone liegt daher bevorzugt im Bereich von 0,2 bis 20 bar, besonders bevorzugt von 0,3 bis 10 bar, ganz besonders bevorzugt von 0,4 bis 5 bar. Bei den vorangehend und im Folgenden aufgeführten Druckangaben handelt es sich - sofern nichts anderes explizit erwähnt - um absolute Druckangaben.

Bevorzugt wird das am Kopf der Umesterungskolonne in dem Verfahren zur Herstellung des Dialkylcarbonates entnommene Dampfgemisch enthaltend Dialkylcarbonat und Alkylalkohol nach Kondensation am Kopf der Umesterungskolonne ganz oder teilweise wenigstens einem weiteren Verfahrensschritt enthaltend wenigstens eine Destillationskolonne zur Trennung von Dialkylcarbonat und Alkylalkohol zugeführt.

Erläuterungen zu den Figuren:

K1 Umesterungskolonne

K2 erste Destillationskolonne zur Trennung des Gemisches enthaltend Dialkylcarbonat und Alkylalkohol

K3 zweite Destillationskolonne zur Trennung des Gemisches enthaltend Dialkylcarbonat und Alkylalkohol

1 Eduktstrom enthaltend Alkylencarbonat und/oder optional Katalysator
2 Eduktstrom enthaltend nahezu reinen Alkylalkohol
3 Eduktstrom enthaltend Alkylalkohol und Dialkylcarbonat
4 Strom enthaltend Alkylenglykol
5 Strom enthaltend aufgereinigtes Dialkylcarbonat
6 Strom enthaltend Dialkylcarbonat und Alkylalkohol
7 Strom enthaltend nahezu reinen Alkylalkohol
8 Strom enthaltend Extraktionsmittel (vorzugsweise Alkylencarbonat)
9 Strom enthaltend Extraktionsmittel (vorzugsweise Alkylencarbonat)
10 Strom enthaltend Extraktionsmittel (vorzugsweise Alkylencarbonat)

**Fig. 1** beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Zweidruckdestillation in einer ersten (K2) und einer zweiten (K3) Destillationskolonne.

**Fig. 2** beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels einer einzelnen Destillationskolonne

(K2).

**Fig. 3** beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Extraktivdestillation in einer ersten (K2) und einer zweiten (K3) Destillationskolonne, wobei das Alkylencarbonat vorzugsweise als Extraktionsmittel verwendet wird.

**Fig. 4** beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Destillation und Dampfpermeation in einer Destillationskolonne (K2).

**Fig. 5** beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol mittels Destillation und Pervaporation in einer Destillationskolonne (K2).

Die folgenden Beispiele dienen der exemplarischen Erläuterung der Erfindung und sind nicht als Beschränkung aufzufassen.

**Beispiele**

Anhand eines Beispiels wird nun die bevorzugte Betriebsweise für das erfindungsgemäße Verfahren detailliert aufgezeigt. Beispiel 1 zeigt die bevorzugte Betriebsweise für die Dialkylcarbonat-Aufreinigungskolonne. Dieses Beispiel soll auf keine Weise als Limitierung der Erfindung ausgelegt werden.

Der Vorteil des erfindungsgemäßen Verfahrens, nämlich die Verringerung der Bildung von unerwünschten Nebenprodukten wie Alkoxyalkohole und aliphatischen Carbonatethern durch die geeignete Einstellung der Verweilzeit der Reaktanden in der flüssigen Phase innerhalb der Aufreinigungskolonne wird mit Hilfe von zwei Vergleichsbeispielen im Folgenden dargestellt.

Sowohl im Beispiel 1 als auch in den Vergleichsbeispielen entstehen Dimethylcarbonat als Dialkylcarbonat und Ethylenglykol aus der Reaktion zwischen Ethylencarbonat und Methanol. Methoxyethanol ist in diesem Falle der Alkoxyalkohol und Methyl-Methoxyethyl-Carbonat (MMEC) der aliphatische Carbonatether.

**Beispiel 1**

Eine Reaktivdestillationskolonne besteht aus einem Verstärkungsteil mit 9 theoretischen Stufen, einer Reaktionszone mit 25 Reaktionsböden (HoldupBoden: 0,6 $m^3$) und einem Abtriebsteil mit 4 theoretischen Stufen. Die Kolonne wird bei einem Druck von 400 mbar (absolut) am Kopf der Kolonne und einem massenbezogenen Rücklaufverhältnis von 0,585 betrieben.

In den oberen Kolonnenbereich direkt oberhalb des ersten Reaktionsbodens werden kontinuierlich 9000 kg/h Ethylencarbonat mit einem Ethylenoxid-Gehalt von 100 ppm und 174 kg/h eines Gemisches mit 33,3 Gew.-% KOH und 66,7 Gew.-% Ethylenglykol zudosiert. Zwischen dem 8. und 9. Reaktionsboden wird der rückgeführte Destillatstrom der Dialkylcarbonat-Aufreinigungskolonne mit einem Massenstrom von 21371 kg/h dampfförnig eingespeist. Zusätzlich dazu werden am unteren Ende der Reaktionszone 7124 kg/h eines Dampfgemisches mit 99,5 Gew.% Methanol, 0,4 Gew.-% Ethylenglykol sowie geringen Mengen an Dimethylcarbonat und anderen Substanzen zugeführt.

Der Sumpfverdampfer wird bei 102 °C betrieben und man erhält 7018 kg/h flüssiges Sumpfprodukt, das hauptsächlich Ethylenglykol beinhaltet.

Ein Partialkondensator kondensiert den Dampfstrom am Kopf der Kolonne bei 40°C. Dadurch werden 6 kg/h dampfförmiges Destillat abgezogen. Das flüssige Destillat mit einem Massenstrom von 30645 kg/h wird zur weiteren Aufreinigung einer weiteren Destillationskolonne zugeführt.

Die Destillationskolonne zur Aufreinigung des bei der Umesterung entstehenden Dialkylcarbonats, bestehend aus einem Verstärkungsteil mit 28 theoretischen Stufen und einem Abtriebsteil mit 11 theoretischen Stufen, wird bei einem Druck von 10 bar (absolut) am Kopf der Kolonne und einem massenbezogenen Rücklaufverhältnis von 1,0 betrieben.

Im unteren Kolonnenbereich werden zwischen der 27. und 28. theoretischen Trennstufe kontinuierlich 30645 kg/h eines Dialkylcarbonat enthaltenden Alkoholgemischs mit 59 Gew.-% Methanol und 41 Gew.-% Dimethylcarbonat zudosiert.

Ein Partialkondensator kondensiert den Dampfstrom am Kopf der Kolonne bei 137 °C. Man erhält sowohl 21 kg/h dampfförmiges Destillat mit einer Zusammensetzung von 82,9 Gew.% Methanol, 14,4 Gew.-% Dimethylcarbonat, 0,3 Gew.-% Ethylenoxid und 2,4 Gew.-% $CO_2$ als auch 21380 kg/h flüssiges Destillat mit einer Zusammensetzung von 84 Gew.-% Methanol und 16 Gew.% Dimethylcarbonat. Zur Vermeidung der Anreicherung leichtsiedender Komponenten wird ein Purgestrom von 9 kg/h dem Destillatstrom entnommen und 21371 kg/h zur Umesterungskolonne zurückgeführt.

Die 1. bis 39. Stufe haben jeweils einen Flüssigkeits-Holdup von 0,06 $m^3$. Der Sumpf der Kolonne hat einen Flüssigkeit-Holdup von 16,5 $m^3$. Die Temperatur der Flüssigkeit im Sumpf der Kolonne beträgt 183°C. Die mittlere Dichte des Flüssigkeit-Holdups beträgt 860 kg/$m^3$. Die mittlere Verweilzeit beträgt 1,6 h.

Man erhält 9244 kg/h flüssiges Sumpfprodukt mit 99,5 Gew.-% Dimethylcarbonat. Neben Methanol finden sich noch 11 ppm Methoxyethanol sowie 5 ppm MMEC.

## Vergleichsbeispiel 1

Es wird der gleiche Aufbau der Kolonnen, wie im Beispiel 1 beschrieben, verwendet. Die Kolonne zur Aufreinigung des Dialkylcarbonates wird bei einem Druck von 10 bar (absolut) am Kopf der Kolonne und einem massenbezogenen Rücklaufverhältnis von 1,0 betrieben.

Im unteren Bereich der Kolonne zur Aufreinigung des Dialkylcarbonates werden zwischen der 27. und 28. theoretischen Trennstufe kontinuierlich 30645 kg/h eines Dialkylcarbonat enthaltenden Alkoholgemischs mit 59 Gew.-% MeOH und 41 Gew.-% Dimethylcarbonat zudosiert.

Ein Partialkondensator kondensiert den Dampfstrom am Kopf der Kolonne bei 137°C. Man erhält sowohl 21 kg/h dampfförmiges Destillat mit einer Zusammensetzung von 83,3 Gew.% Methanol, 14,6 Gew.-% Dimethylcarbonat, 0,3 Gew.% Ethylenoxid und 1,8 Gew.-% CO2 als auch 21380 kg/h flüssiges Destillat mit einer Zusammensetzung von 84 Gew.-% Methanol und 16 Gew.% Dimethylcarbonat. Zur Vermeidung der Anreicherung leichtsiedender Komponenten wird ein Purgestrom von 9 kg/h dem Destillatstrom entnommen und 21371 kg/h zur Umesterungskolonne zurückgeführt.

Die 1. bis 39. Stufe haben jeweils einen Flüssigkeits-Holdup von 0,3 $m^3$. Der Sumpf der Kolonne hat einen Flüssigkeit-Holdup von 25 $m^3$. Die Temperatur der Flüssigkeit im Sumpf der Kolonne beträgt 183 °C. Die mittlere Dichte des Flüssigkeit-Holdups beträgt 860 kg/$m^3$. Die mittlere Verweilzeit beträgt 2,6 h.

Man erhält 9244 kg/h flüssiges Sumpfprodukt mit 99,5 Gew.-% Dimethylcarbonat. Neben Methanol finden sich noch 38 ppm Methoxyethanol sowie 24 ppm MMEC.

## Vergleichsbeispiel 2

Es wird der gleiche Aufbau der Kolonnen, wie im Beispiel 1 beschrieben, verwendet. Die Kolonne zur Aufreinigung des Dialkylcarbonates wird jedoch bei einem Druck von 20 bar (absolut) am Kopf der Kolonne und einem Rücklaufverhältnis von 1,0 betrieben.

Die Erhöhung des Betriebsdrucks der Dialkylcarbonat-Aufreinigungskolonne und die Druckabhängigkeit der Zusammensetzung des Methanol/Dimethylcarbonat-Azeotrops führt auf geänderte Betriebsbedingungen in der Umesterungskolonne, die im Folgenden aufgeführt werden.

Die Umesterungskolonne wird bei einem Druck von 400 mbar (absolut) am Kopf der Kolonne und einem massenbezogenen Rücklaufverhältnis von 0,585 betrieben. In den oberen Kolonnenbereich direkt oberhalb des ersten Reaktionsbodens werden kontinuierlich 9000 kg/h Ethylencarbonat mit einem Ethylenoxid-Gehalt von 100 ppm und 174 kg/h eines Gemisches mit 33,3 Gew.-% KOH und 66,7 Gew.% Ethylenglykol zudosiert. Zwischen dem 8. und 9. Reaktionsboden wird der rückgeführte Destillatstrom der Dialkylcarbonat-Aufreinigungskolonne mit einem Massenstrom von 21371 kg/h und einer Zusammensetzung von 90,5 Gew.-% Methanol und 9,5 Gew.% Dimethylcarbonat dampffömig eingespeist. Zusätzlich dazu werden am unteren Ende der Reaktionszone 7124 kg/h eines Dampfgemisches mit 99,5 Gew.% Methanol, 0,4 Gew.-% Ethylenglykol sowie geringen Mengen an Dimethylcarbonat und anderen Substanzen zugeführt. Der Sumpfverdampfer wird bei 102 °C betrieben und man erhält 7018 kg/h flüssiges Sumpfprodukt, das hauptsächlich Ethylenglykol beinhaltet. Ein Partialkondensator kondensiert den Dampfstrom am Kopf der Kolonne bei 40°C. Dadurch werden 6 kg/h dampfförmiges Destillat abgezogen. Das flüssige Destillat mit einem Massenstrom von 30645 kg/h wird zur weiteren Aufreinigung der Dialkylcarbonat-Aufreinigungskolonne zugeführt.

Analog zu Beispiel 1 und Vergleichsbeispiel 1 besteht die Destillationskolonne zur Aufreinigung des bei der Umesterung entstehenden Dialkylcarbonats aus einem Verstärkungsteil mit 28 theoretischen Stufen und einem Abtriebsteil mit 11 theoretischen Stufen. Die Aufreinigungskolonne wird bei einem Druck von 20 bar (absolut) am Kopf der Kolonne und einem massenbezogenen Rücklaufverhältnis von 1,0 betrieben.

Im unteren Bereich der Kolonne zur Aufreinigung des Dialkylcarbonates werden zwischen der 27. und 28. theoretischen Trennstufe kontinuierlich 30645 kg/h eines Dialkylcarbonat enthaltenden Alkoholgemischs mit 63,4 Gew.-% MeOH und 36,6 Gew.% Dimethylcarbonat zudosiert.

Ein Partialkondensator kondensiert den Dampfstrom am Kopf der Kolonne bei 167°C. Man erhält sowohl 21

kg/h dampfförmiges Destillat mit einer Zusammensetzung von 91,4 Gew.-% Methanol, 7,7 Gew.-% Dimethylcarbonat, 0,7 Gew.% Ethylenoxid und 0,2 Gew.-% CO2 als auch 21374 kg/h flüssiges Destillat mit einer Zusammensetzung von 90,5 Gew.-% Methanol und 9,5 Gew.% Dimethylcarbonat. Zur Vermeidung der Anreicherung leichtsiedender Komponenten wird ein Purgestrom von 3 kg/h dem Destillatstrom entnommen und 21371 kg/h zur Umesterungskolonne zurückgeführt.

Die 1. bis 39. Stufe haben jeweils einen Flüssigkeits-Holdup von 0,3 $m^3$. Der Sumpf der Kolonne hat einen Flüssigkeit-Holdup von 25 $m^3$. Die Temperatur der Flüssigkeit im Sumpf der Kolonne beträgt 224 °C. Die mittlere Dichte des Flüssigkeit-Holdups beträgt 750 kg/$m^3$. Die mittlere Verweilzeit beträgt 2,3 h.

Man erhält 9250 kg/h flüssiges Sumpfprodukt mit 99,5 Gew.-% Dimethylcarbonat. Neben Methanol finden sich noch 53 ppm Methoxyethanol sowie 111 ppm MMEC.

## Patentansprüche

1. Verfahren zur Aufreinigung von Dialkylcarbonat der Formel (II)

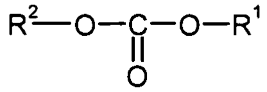

(II),

in der $R^1$ und $R^2$ unabhängig voneinander für lineares oder verzweigtes, substituiertes oder unsubstituiertes $C_1$-$C_6$-Alkyl stehen, in einer oder mehreren Kolonnen in Gegenwart eines Alkylenoxids der Formel (V)

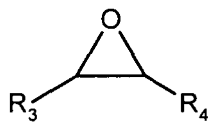

(V)

wobei in der Formel $R^3$ und $R^4$ unabhängig voneinander Wasserstoff, substituiertes oder nicht substituiertes $C_1$-$C_4$-Alkyl, substituiertes oder nicht substituiertes $C_2$-$C_4$-Alkenyl oder substituiertes oder nicht substituiertes $C_6$-$C_{12}$-Aryl bedeutet,
und eines Alkylalkohols der Formel (IV)

$R^5$-OH (IV),

wobei $R^5$ ein geradkettiges oder verzweigtes $C_1$-$C_4$-Alkyl bedeutet,
**dadurch gekennzeichnet, dass**
die mittlere Verweilzeit des Dialkylcarbonates in der oder den Kolonne(n) im Bereich von 0,3h bis 2h liegt.

2. Verfahren nach Anspruch 1, **dadurch gekennzeichnet, dass** die mittlere Verweilzeit des Dialkylcarbonates in der oder den Kolonne(n) im Bereich von 0,5h bis 2h liegt.

3. Verfahren nach Anspruch 1 oder 2, **dadurch gekennzeichnet, dass** als Alkohol Methanol oder Ethanol verwendet wird.

4. Verfahren nach einem der Ansprüche 1 bis 3, **dadurch gekennzeichnet, dass** Alkylen für Ethylen oder Propylen steht.

5. Verfahren nach einem der Ansprüche 1 bis 4, **dadurch gekennzeichnet, dass** der Druck in wenigstens einer zur Aufreinigung des Dialkylcarbonats eingesetzten Kolonnen im Bereich von $0{,}5 \cdot 10^5$ Pa bis $50 \cdot 10^5$Pa (0,5 bis 50 bar) absolut liegt.

6. Verfahren nach Anspruch 5, **dadurch gekennzeichnet, dass** der Druck in wenigstens einer zur Aufreinigung des Dialkylcarbonats eingesetzten Kolonnen im Bereich von $2 \cdot 10^5$ Pa bis $20 \cdot 10^5$ Pa (2 bis 20 bar) absolut liegt

7. Verfahren nach einem der Ansprüche 1 bis 6, **dadurch gekennzeichnet, dass** die Temperatur in wenigstens einer

zur Aufreinigung des Dialkylcarbonats eingesetzten Kolonnen im Bereich von 120°C bis 210°C liegt.

**8.** Verfahren nach einem der Ansprüche 1 bis 7, **dadurch gekennzeichnet, dass** wenigstens eine der zur Aufreinigung des Dialkylcarbonats eingesetzten Kolonnen eine Packungskolonne ist.

## Claims

**1.** Process for purifying dialkyl carbonate of the formula (II)

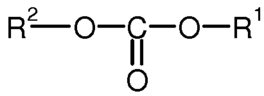

(II)

in which $R^1$ and $R^2$ are each independently linear or branched, substituted or unsubstituted $C_1$-$C_6$-alkyl in one or more columns in the presence of an alkylene oxide of the formula (V)

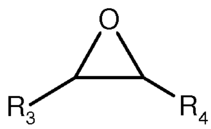

(V)

where $R^3$ and $R^4$ in the formula are each independently hydrogen, substituted or unsubstituted C1-$C_4$-alkyl, substituted or unsubstituted $C_2$-$C_4$-alkenyl or substituted or unsubstituted $C_6$-$C_{12}$-aryl,
and of an alkyl alcohol of the formula (IV)

$R^5$-OH (IV)

where $R^5$ is a straight-chain or branched $C_1$-$C_4$-alkyl,
**characterized in that**
the mean residence time of the dialkyl carbonate in the column(s) is in the range from 0.3 h to 2 h.

**2.** Process according to Claim 1, **characterized in that** the mean residence time of the dialkyl carbonate in the column(s) is in the range from 0.5 h to 2 h.

**3.** Process according to Claim 1 or 2, **characterized in that** the alcohol used is methanol or ethanol.

**4.** Process according to any of Claims 1 to 3, **characterized in that** alkylene is ethylene or propylene.

**5.** Process according to any of Claims 1 to 4, **characterized in that** the pressure in at least one of the columns used for purification of the dialkyl carbonate is in the range from $0.5.10^5$ Pa to.$50.10^5$ Pa (0.5 to 50 bar) absolute.

**6.** Process according to Claim 5, **characterized in that** the pressure in at least one of the columns used for purification of the dialkyl carbonate is in the range from $2.10^5$ Pa to $20.10^5$ Pa (2 to 20 bar) absolute.

**7.** Process according to any of Claims 1 to 6, **characterized in that** the temperature in at least one of the columns used for purification of the dialkyl carbonate is in the range from 120°C to 210°C.

**8.** Process according to any of Claims 1 to 7, **characterized in that** at least one of the columns used for purification of the dialkyl carbonate is a packed column.

## Revendications

**1.** Procédé de purification de carbonate de dialkyle de formule (II)

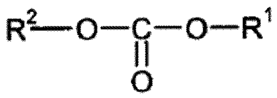

(II),

dans laquelle $R^1$ et $R^2$ représentent indépendamment l'un de l'autre un alkyle en $C_1$-$C_6$ linéaire ou ramifié, substitué ou non substitué, dans une ou plusieurs colonnes en présence d'un oxyde d'alkylène de formule (V)

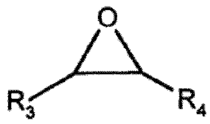

(V)

dans la formule, $R^3$ et $R^4$ signifiant indépendamment l'un de l' autre l'hydrogène, un alkyle en $C_1$-$C_4$ substitué ou non substitué, un alcényle en $C_2$-$C_4$ substitué ou non substitué, ou un aryle en $C_6$-$C_{12}$ substitué ou non substitué,
et un alcool alkylique de formule (IV)

$R^5$-OH (IV)

dans laquelle $R^5$ signifie un alkyle en $C_1$-$C_4$ linéaire ou ramifié,
**caractérisé en ce que**
le temps de séjour moyen du carbonate de dialkyle dans la ou les colonnes se situe dans la plage allant de 0,3 h à 2 h.

**2.** Procédé selon la revendication 1, **caractérisé en ce que** le temps de séjour moyen du carbonate de dialkyle dans la ou les colonnes se situe dans la plage allant de 0,5 h à 2 h.

**3.** Procédé selon la revendication 1 ou 2, **caractérisé en ce que** le méthanol ou l'éthanol est utilisé en tant qu'alcool.

**4.** Procédé selon l'une quelconque des revendications 1 à 3, **caractérisé en ce que** l'alkylène représente l'éthylène ou le propylène.

**5.** Procédé selon l'une quelconque des revendications 1 à 4, **caractérisé en ce que** la pression dans au moins une des colonnes utilisées pour la purification du carbonate de dialkyle se situe dans la plage allant de $0,5 \cdot 10^5$ Pa à $50 \cdot 10^5$ Pa (0,5 à 50 bar) absolu.

**6.** Procédé selon la revendication 5, **caractérisé en ce que** la pression dans au moins une des colonnes utilisées pour la purification du carbonate de dialkyle se situe dans la plage allant de $2 \cdot 10^5$ Pa à $20 \cdot 10^5$ Pa (2 à 20 bar) absolu.

**7.** Procédé selon l'une quelconque des revendications 1 à 6, **caractérisé en ce que** la température dans au moins une des colonnes utilisées pour la purification du carbonate de dialkyle est de 120 °C à 210 °C.

**8.** Procédé selon l'une quelconque des revendications 1 à 7, **caractérisé en ce qu'**au moins une des colonnes utilisées pour la purification du carbonate de dialkyle est une colonne à garnissage.

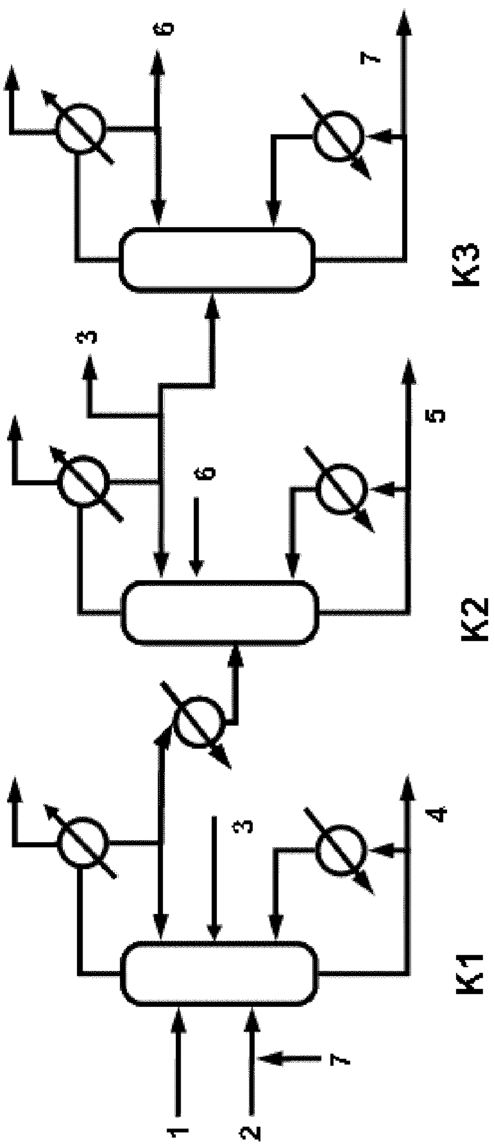


Fig. 1

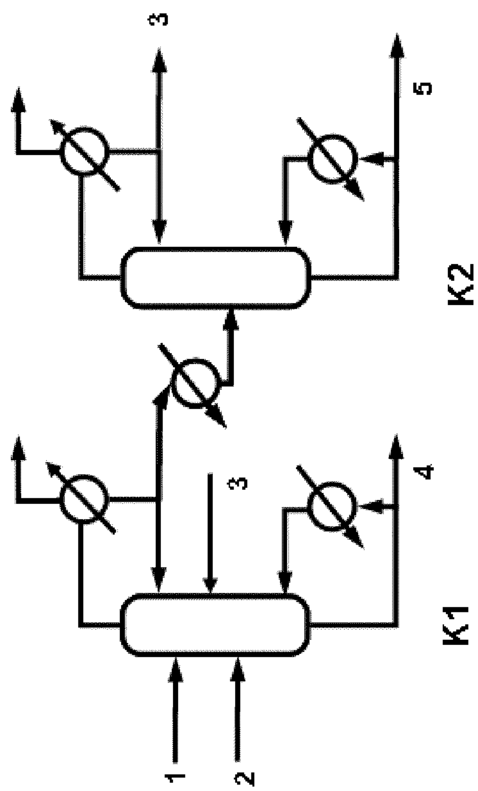


Fig. 2

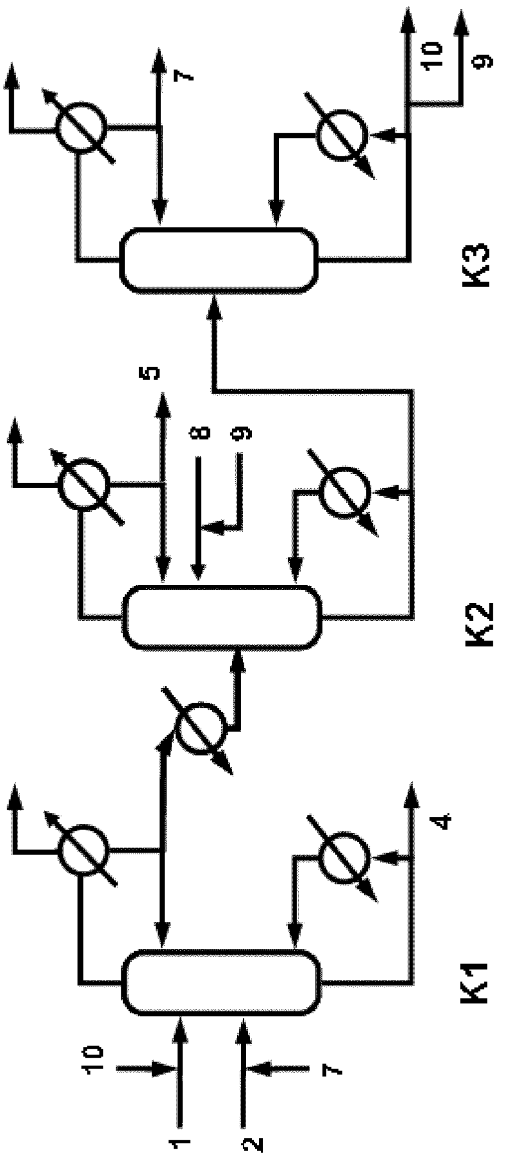


Fig. 3

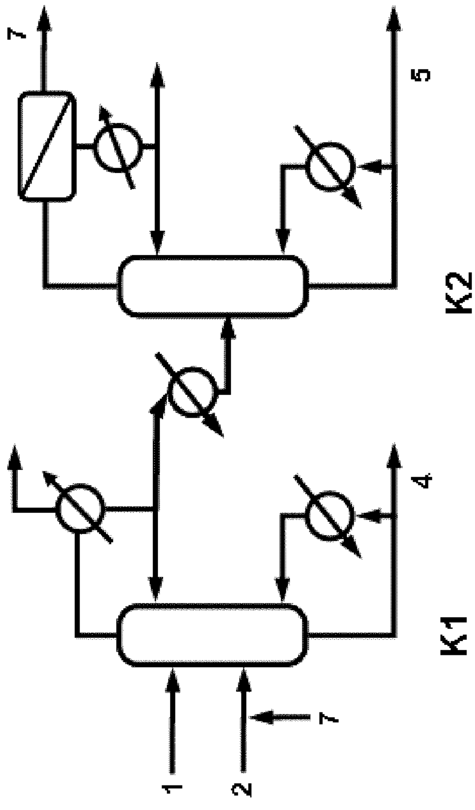


Fig. 4

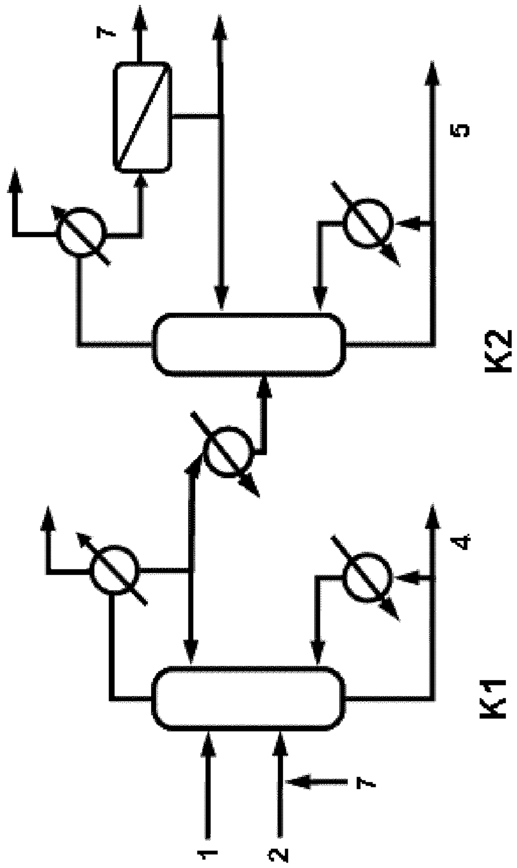


Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6930195 B2
- EP 530615 A1
- EP 569812 A1
- EP 1086940 A1
- EP 1760059 A1
- EP 1967242 A1
- EP 1967508 A1
- EP 1961731 A
- US 4162200 A
- EP 581115 B1
- EP 592883 B1
- WO 2007096343 A1


### In der Beschreibung aufgeführte Nicht-Patentliteratur


- Ullmann's Encyclopädie der Technischen Chemie. vol. 2, 528 ff
- Ullmann's Encyclopedia of Industrial Chemistry. Chemie Ingenieur Technik. 2007, vol. 7, 11, , 95